# EUROPEAN PATENT APPLICATION

(11) **EP 3 395 326 A1**
(43) Date of publication of application: **31.10.2018**
(21) Application number: 17167773.5
(22) Date of filing: 24.04.2017
(51) Int. Cl.: A61K 9/00, A61K 47/34, C08J 3/075, C08L 71/02

(54) **HYDROGEL AND HYDROGEL-FORMING COMPOSITIONS WITH DUAL GELATION MECHANISM**

(71) Applicant: Universität Regensburg, 93053 Regensburg (DE)
(72) Inventor: GREGORITZA, Manuel, 85464 Finsing (DE); GÖPFERICH, Achim, 93161 Sinzing (DE); BRANDL, Ferdinand, 76135 Karlsruhe (DE)
(74) Representative: Vossius & Partner Patentanwälte Rechtsanwälte mbB

(57) **Abstract**

The present invention relates to a composition comprising a plurality of molecules of a polymer A and a plurality of molecules of a polymer B, wherein polymer A carries two or more diene groups bound to the polymer structure as pendant reactive groups for a Diels-Alder reaction with polymer B; polymer B carries two or more dienophile groups bound to the polymer structure as pendant reactive groups for a Diels-Alder reaction with polymer A; and wherein at least one of polymer A and polymer B is a temperature-responsive polymer.

## Description

Due to their versatility and many attractive properties, hydrogels have numerous applications in pharmaceutics (Peppas, N. Eur. J. Pharm. Biopharm. 2000, 50 (1), 27-46; Buwalda, S. J et al. Biomacromolecules 2017, 18 (2), 316-330), regenerative medicine (Slaughter, B. V. et al., Adv. Mater. 2009, 21, 3307-3329; Yang, J.-A. et a!., Prog. Po/ym. Sci. 2014, 39 (12), 1973-1986), and biomedical engineering (Klouda, L.; Mikos, A. G., Eur. J. Pharm. Biopharm. 2008, 63 (1), 34-45; Gaharwar, A. K. et al., A. Biotechnol. Bioeng. 2013, 111 (3), 441-453; Jiang, Y. et al., Z. Biomaterials 2014, 35 (18), 4969-4985). In drug delivery, hydrogels are frequently used as carrier systems for therapeutic antibodies and other biopharmaceuticals as they are deemed biocompatible and able to preserve the activity of the incorporated proteins (Vermonden, T. et a!., Chem. Rev. 2012, 112 (5), 2853-2888). Moreover, they can accommodate high loads of peptides and proteins e.g. by *in situ* encapsulation (Lin, C.-C.; Anseth, K. S. Pharm. Res. 2008, 26 (3), 631-643).

The Diels-Alder (DA) reaction presents an attractive cross-linking reaction for hydrogels. The DA reaction proceeds in water, reaction kinetics are independent from pH, the reaction does not require any catalysts and does not lead to the formation of toxic side products (Gregoritza, M.; Brandl, F. P. Eur. J. Pharm. Biopharm. 2015, 97, 438-453). To that extent, the DA reaction between maleimide and furan has been used as a cross-linking mechanism for the preparation of hydrogels incorporating active agents, e.g. for antibody delivery (Kirchhof, S. et al., F. P. Eur. J. Pharm. Biopharm. 2015, 96, 217-225; Gregoritza, M. et al. J. Mater. Chem. B 2016, 4, 3398-3408; WO 2013/068397 A1). However, relatively long gelling times were observed for these hydrogels. This is a potential drawback, as it delays gel formation upon administration.

An alternative approach for the preparation of hydrogels is to use materials that respond to external stimuli, such as temperature, pH or ionic strength (Hoffman, A. S. Adv. Drug Deliv. Rev. 2013, 65 (1), 10-16; He, C. et al., J. Control. Release 2008, 127 (3), 189-207; Koetting, M. C. et al., Mater. Sci. Eng., R 2015, 93, 1-49; Qiu, Y.; Park, K. Adv. Drug Deliv. Rev. 2012, 64 (S), 49-60). For example, hydrogels can be prepared using thermoresponsive block copolymers, e.g., poly(N-isopropylacrylamide) (pNIPAM), poloxamer (Pluronic^{®}), and poloxamine (Tetronic^{®}) (Ruel-Gariépy, E.; Leroux, J.-C. Eur. J. Pharm. Biopharm. 2004, 58 (2), 409-426). These polymers exhibit a reduction in solubility above a certain temperature leading to gel formation. A drawback of these hydrogels, however, is their low resistance against dissolution.

Hydrogels combining thermal gelation and covalent cross-linking have been provided using Michael-type additions between acrylate and thiol groups (Cellesi, F. et al., Macromol. Chem. Phys. 2002, 203, 1466-1472; Cellesi, F., et al. Biomaterials 2004, 25 (21), 5115-5124; Cellesi, F.; Tirelli, N. J. Mater. Sci. 2005, 16, 559-565; Cho, E.et al., Acta Biomaterialia 2012, 8 (6), 2223-2232; Censi, R. et al., Macromolecules 2010, 43 (13), 5771-5778; Pritchard, C.D. et al., Biomaterials 2011, 32, 587-597). However, also Michael-type additions between thiol and acrylate groups have some disadvantages. Thus, due to the high rate at which the addition reaction and the resulting gelation proceeds, compositions containing compounds which can react via a Michael-type reaction tend to be difficult to administer to a patient. For example, Pritchard et al. (loc. cit.) report a gelation time in a neutral environment at 25 °C of about 100 s. Moreover, thiol groups on polymer chains can form dimers, and if polymers with thiol functional groups are used for formulating proteins as active agents, they may alter the structure of proteins through thiol-disulfide exchange. Furthermore, thiol-Michael additions are often conducted in a basic environment to accelerate the reaction, which makes them less suitable for use with active agents that are formulated in an acidic buffer, such as a variety of antibodies, e.g., adalimumab (pH 5.2), ranibizumab (pH 5.5), trastuzumab (pH 6.0), and Bevacizumab (pH 6.2).

In the context of the present invention, hydrogels and hydrogel-forming compositions are provided which rely on the gelation of polymers used as macromonomers which combine temperature-responsive properties and functional groups suitable for a Diels-Alder (DA) reaction. Their gelation involving a thermal gelation and a Diels-Alder reaction in a dual gelation process allows hydrogels to be provided which gel rapidly when exposed to an increase in temperature, which have a satisfactory strength or durability due to the presence of covalent bonds formed via the Diels-Alder reaction, e.g. to deliver an active agent incorporated therein over extended periods of time, but which are nevertheless degradable.

Thus, the invention provides products suitable for the preparation of a hydrogel, in particular a composition or a kit, comprising a plurality of molecules of a polymer A and a plurality of molecules of a polymer B, wherein polymer A carries two or more diene groups bound to the polymer as pendant reactive groups for a Diels-Alder reaction with polymer B; polymer B carries two or more dienophile groups bound to the polymer as pendant reactive groups for a Diels-Alder reaction with polymer A; and wherein at least one of polymer A and polymer B is a temperature-responsive polymer. Moreover, the invention provides a hydrogel which is obtainable via gelation of a composition comprising the polymers A and polymers B as macromonomers, said gelation involving the thermal gelation of the composition comprising a plurality of molecules of a polymer A and a plurality of molecules of a polymer B, and the Diels-Alder reaction of the diene reactive groups of polymer A with the dienophile reactive groups of polymer B.

The hydrogel-forming compositions in accordance with the invention combine thermal gelation and DA chemistry in a dual gelation process to provide the hydrogels in accordance with the invention. As will be understood by the skilled reader, the gelation to form these hydrogels proceeds via two processes which proceed on different timescales, i.e. the - relatively fast- thermal gelation of the temperature-responsive polymer(s) which can be triggered via an increase in temperature, and the -relatively slower- gelation due to the formation of covalent bonds between polymer A and polymer B via the Diels-Alder reaction of the diene groups and the dienophile groups which already begins after mixing polymer A with polymer B.

The hydrogels and the hydrogel-forming compositions in accordance with the invention that combine thermal gelation and DA chemistry in a dual gelation process provide advantages especially for pharmaceutical application, e.g. the delivery of active agents such as therapeutic antibodies from a hydrogel matrix. Precursor compositions can easily be prepared e.g. by dissolving the polymers used as macromonomers in a liquid carrier, such as water or water containing a buffer, together with the active agent to be delivered. Due to the reaction kinetics of the DA reaction, the fluidity of the polymer solution will remain unaffected at room temperature for a time span long enough to allow convenient handling and administration thereof. For example, if the polymer carrying a diene group and the polymer carrying a dienophile group are mixed at room temperature, they can be conveniently co-administered using a syringe without the risk of a gel formation in the syringe which would impede the administration. On the other hand, the temperature-responsiveness will lead to rapid gel formation via a physical thermal gelation process if the precursor composition is exposed to higher temperatures, e.g. to body temperature. In that way, a therapeutic agent can be effectively and rapidly incorporated into a hydrogel matrix, and a loss of active agent is avoided, while a sufficient stability of the matrix can be ensured by the additional formation of covalent bonds via DA reaction. It has been found that, even after the thermal gelation, the DA reaction (or chemical gelation) proceeds sufficiently to ensure additional stabilization of the physically gelled material. In contrast to hydrogels that rely exclusively on thermal gelation, these hydrogels are expected to be significantly more stable concerning degradation as a result of covalent bonds formed between the polymers functioning as macromonomers. The stability, and thus e.g. the rate of delivery of a therapeutic agent, can be tailored by adjusting the polymer structure of polymers A and B, and/or by adjusting the number of the pendant functional groups for the DA reaction bound to the polymer. This can also be conveniently achieved by the use of mixtures of polymers A with different structures and/or different numbers of pendant functional groups, and/or the use of mixtures of polymers B with different structures and/or different numbers of pendant functional groups.

### Polymers A and B

In accordance with the present invention, the polymers A and B provide reactive groups which are capable of undergoing a Diels-Alder reaction, in particular mutually reactive groups in the sense that the reactive groups of polymer A are capable of undergoing the Diels-Alder reaction with the reactive groups of polymer B. Specifically, polymer A carries two or more diene groups bound to the polymer structure as pendant reactive groups for a Diels-Alder reaction with polymer B, and polymer B carries two or more dienophile groups bound to the polymer structure as pendant reactive groups for a Diels-Alder reaction with polymer A. To that extent, the molecules of polymer A and of polymer B are also referred to herein as "macromonomers", since their reaction provides the hydrogel polymer in accordance with the invention as a polymer containing a plurality of monomeric units provided by the polymers A and B.

As will be understood by the skilled person, the Diels-Alder reaction as referred to herein represents a [4+2]-cycloaddition reaction between a diene group and a dienophile group. Typically and preferably, dienophiie groups are used which have a C-C double bond. However, compounds can be reacted as dienophiles in a Diels-Alder reaction which have a double bond between a carbon atom and a heteroatom (e.g. N, O or S) or between two heteroatoms (e.g. N-N or N-O), such as e.g. an aldehyde, a ketone, an imine or a thioketone. A typical exemplary Diels-Alder (DA) reaction that can be used for the purpose of the present invention is represented by the following reaction equation.

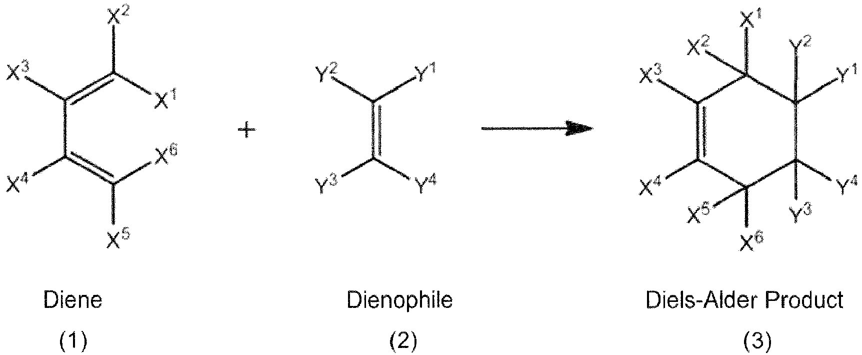

The equation shows a general schematic of DA [4+2]-cycloaddition between a diene of the formula (1) and a dienophile of the formula (2), forming a Diels-Alder product of the formula (3). The groups X¹ to X⁶ and Y¹ to Y⁴ represent any desired atoms or molecule groups. Suitable pairs of X¹ to X⁶ and Y¹ to Y⁴, respectively, may be combined to form a ring structure.

In the context of the present invention, the diene and the dienophile is bound as a reactive group to the polymer structure of polymer A and the polymer B, respectively. Thus, one of X¹ to X⁶ and Y¹ to Y⁴ provides the attachment of the diene or dienophile to the polymer structure and typically represents a bond or a divalent linking group.

The DA reaction takes place at an accelerated rate in an aqueous medium; the addition of catalysts or initiators is not required. By suitably selecting the substituents X¹ to X⁶ and Y¹ to Y⁴, the rate of the reaction can be influenced. In order to accelerate the reaction, electron-donating substituents (groups with +i and/or +M-effect, e.g. alkyl groups) are selected for X¹ to X⁶; electron-withdrawing substituents (groups with -I and/or -M-effect) are preferably used for Y¹ to Y⁴. For example, dienes of the formula (1) can independently carry hydrogen atoms, hydrocarbon groups (such as e.g. alkyl groups), or alkoxy groups (in particular methoxy groups) as groups X¹ to X⁶. Furthermore, in particular the groups X¹ and X⁶ can be linked to each other, thus forming a carbocyclic or heterocyclic five- or six-membered ring system, such as a furan ring, a cyclopentadiene ring or a 1,3-cyclohexadiene ring.

Dienophiles of the formula (2) can for example carry a hydrogen atom or a hydrocarbon group, e.g. an alkyl group, at two or three of the positions Y¹ to Y⁴ and at the remaining position(s) an electron-withdrawing group such as an aldehyde function -C(O)H, a keto function -C(O)R, an ester function -C(O)OR, a cyano group or a nitro group, wherein R is a hydrocarbon group, e.g. an alkyl group. Also well suited as dienophiles are compounds of the formula (2) wherein Y² and Y³ represent a hydrogen atom or a hydrocarbon group, e.g. an alkyl group, and Y¹ and Y⁴ are linked to each other by forming a maleic acid anhydride, a maleimide or a 1,4-quinone.

The Diels-Alder reaction does not lead to the release of low-molecular and potentially toxic byproducts. Furthermore, undesired side reactions with other functional groups can largely be excluded. This is due to the orthogonality of the functional groups used in the reaction (diene and dienophile). These functional groups are highly selective in their reactivity, and can be used in combination with a broad variety of other functional groups (e.g. alcohols, carboxylic acids, etc.) without giving rise to undesired side reactions; the use of protecting groups is not necessary. This is of special value for the use of the hydrogels of the present invention for the delivery of active agents, since uncontrolled and therefore activity-decreasing reactions with functional groups of active agents, e.g. with therapeutically active peptides, proteins or nucleic acids, can be eliminated. In addition to the advantages already mentioned, the dienes and dienophile also show excellent storage stability under suitable conditions, e.g. compared to activated carboxylic acids or thiols used in other types of coupling reactions.

The diene groups bound to the polymer structure of polymer A are as a rule open chain or cyclic groups which have two conjugated C-C double bonds. The double bonds in open chain compounds are present in a cisoid conformation. Examples of such dienes include those of formula (1) above. Preferred diene groups bound to the polymer structure of polymer A are selected from a furane moiety, a cyclopentadiene moiety and a cyclohexadiene moiety. Particularly preferred is a furane moiety.

The dienophile groups bound to the polymer structure of polymer B are preferably compounds with a single C-C double bond or a non-conjugated double bond. Examples of such dienophiles include those of formula (2) above. However, compounds which have a double bond between a carbon atom and a heteroatom (e.g. N, O or S) or between two heteroatoms (e.g. N-N or N-O), such as e.g. an aldehyde, a ketone, an imine or a thioketone, can also be used as dienophiles in a Diels-Alder reaction. Heterocyclic compounds such as 4-phenyl-1,2,4-triazole-3,5-dione (PTAD) can be used as dienophiles as well. In the context of the present invention, the dienophile groups bound to polymer B are more preferably selected from a maleic anhydride moiety, a maleimide moiety, and a 1,4-quinone moiety, and the maleimide moiety is particularly preferred.

Thus, it will be apparent that it is also preferred in the context of the invention that polymer A carries two or more diene groups bound to the polymer structure as pendant reactive groups which are selected from a furane moiety, a cyclopentadiene moiety and a cyclohexadiene moiety, and which are more preferably furane moieties, and polymer B carries two or more dienophile groups bound to the polymer structure as pendant reactive groups for a Diels-Alder reaction with polymer A which are selected from a maleic anhydride moiety, a maleimide moiety, and a 1,4-quinone moiety, and which are more preferably maleimide moieties.

As will be understood by the skilled person, in order to ensure a convenient handling and storage of the polymer A and B, it is preferred that polymer B carries no diene groups, and that polymer A carries no dienophile groups bound to the polymer structure.

As noted above, polymer A carries two or more diene groups bound to its polymer structure as pendant reactive groups, and polymer B carries two or more dienophile groups bound to its polymer structure as pendant reactive groups. As will be understood by the skilled reader, the number of these reactive groups in polymer A and polymer B is indicated as the number of diene groups per molecule of polymer A and the number of dienophile groups per molecule of polymer B, respectively.

It is preferred that at least one of polymer A and polymer B carries three or more of the reactive groups for a Diels-Alder reaction, and more preferred that at least one of polymer A and polymer B carries four or more of the reactive groups for a Diels-Alder reaction. In other words, it is preferred that polymer A carries three or more diene groups, more preferred four or more diene groups, or that polymer B carries three or more dienophile groups, more preferred four or more dienophile groups. It is even more preferred that polymer A carries three or more diene groups, most preferably four or more diene groups, and polymer B carries three or more dienophile groups, most preferably four or more dienophile groups.

The upper limit of the number of diene groups in polymer A is preferably 16, more preferably 12, and most preferably 8. The upper limit of the number of dienophile groups in polymer B is preferably 16, more preferably 12, and most preferably 8.

Thus, it will be apparent that it is also preferred in the context of the invention that polymer A carries 3 to 12, more preferably 4 to 8, diene groups bound to the polymer structure as pendant reactive groups which are selected from a furane moiety, a cyclopentadiene moiety and a cyclohexadiene moiety, and which are more preferably furane moieties, and polymer B carries 3 to 12, more preferably 4 to 8, dienophile groups bound to the polymer structure as pendant reactive groups for a Diels-Alder reaction with polymer A which are selected from a maleic anhydride moiety, a maleimide moiety, and a 1,4-quinone moiety, and which are more preferably maleimide moieties.

The diene groups are bound to the polymer structure of polymer A and the dienophile groups are bound to the polymer structure of polymer B as pendant reactive groups. As used herein, and unless specifically indicated otherwise, the term "polymer structure" encompasses the possibility that the polymer is a copolymer. Reference to the "polymer structure" or the "copolymer structure" of polymer A and polymer B is made herein to underline that the polymers A and B comprise the structure which is formed by their repeating units, i.e. the polymer structure or the copolymer structure, and additionally comprise the diene and dienophil groups as pendant groups, respectively. These pendant groups are generally grafted to the polymer structure by a covalent bond. The diene and the dienophile groups may be bonded directly to the polymer structure, or may be bound via a linker.

In order to allow a hydrogel with a we!! defined polymer architecture to be formed from polymers A and B, it is preferred that the diene groups are bound to the termini, more preferably to each terminus, of the polymer chain or polymer chains of polymer A, and that the dienophile groups are bound to the termini, more preferably to each terminus, of the polymer chain or polymer chains of polymer B. As will be understood by the skilled person, a terminus of a polymer chain is generally formed by the terminal repeating unit of the chain. It is further preferred in this context that no additional diene or dienophile groups, respectively, are bound to the polymer A or polymer B at other positions then the termini along the polymer chain or polymer chains.

A controlled spatial distribution of the diene groups bound to the molecules of polymer A and the dienophile groups bound to the molecules of polymer B can be advantageously used to control the mesh size of the hydrogel, e.g. to adjust the mesh size to the size of molecules of a macromolecular active agent or the size of particles of an active agent to be incorporated into the hydrogel. This can also be conveniently accomplished by binding the diene groups to termini of polymer A and the dienophile groups to the termini of polymer B. In this case, the mesh size of the hydrogel can be controlled via a variation of the length of the polymer chains.

As noted above, it is preferred that polymer A carries three or more, more preferably four or more diene groups. This can be conveniently accomplished e.g. by a linear polymer A which carries two or more diene groups bound to each terminus. More preferably, this is accomplished by using a branched polymer as polymer A which carries one or more, e.g. two, diene groups bound to the termini of the polymer chains, most preferably to the terminus of each polymer chain.

Similarly, it is preferred that polymer B carries three or more, more preferably four or more dienophile groups. This can be conveniently accomplished e.g. by a linear polymer B which carries two or more dienophile groups bound to the termini, preferably bound to each terminus. More preferably, this is accomplished by using a branched polymer as polymer B which carries one or more, e.g. two, dienophil groups bound to the termini of the polymer chains, most preferably to the terminus of each polymer chain.

As will be understood by the skilled reader, in the case of a branched polymer, more than two termini will be provided in the polymer, e.g. at the end of the main chain and of any side chain present. If two or more diene or dienophile groups, respectively, are to be bound to one polymer chain terminus, this can be conveniently accomplished using a di- or higher valent linker which is bound at one valency to the terminal repeating unit in the chain, and provides further valencies for two or more diene or dienophil groups, respectively.

Thus, it will be apparent that it is also preferred in the context of the invention that polymer A is a branched polymer carrying a total of 3 to 12, more preferably 4 to 8, diene groups per molecule wherein one or two diene groups are bound to the terminus of each polymer chain as pendant reactive groups, and the diene groups are selected from a furane moiety, a cyclopentadiene moiety and a cyclohexadiene moiety, and are more preferably furane moieties, and
polymer B is a branched polymer carrying a total of 3 to 12, more preferably 4 to 8, dienophile groups per molecule wherein one or two dienophil groups are bound to the terminus of each polymer chain as pendant reactive groups, and the dienophil groups are selected from a maleic anhydride moiety, a maleimide moiety, and a 1,4-quinone moiety, and are more preferably maleimide moieties.

In the context of the invention, at least one of polymer A and polymer B, preferably both polymer A and polymer B are a temperature-responsive polymer (also referred to as thermo-responsive polymer). In the context of the invention, temperature responsive polymers are generally used which are soluble in water and which show temperature responsive characteristics in water as a solvent, such that they form a gel when the temperature of their solution in water is increased. Preferably, the temperature-responsive polymer provides a solution in water (e.g. at a concentration within the range of 5 to 50 wt%, preferably 10 to 40 wt%, more preferably of 25 to 35 wt% and most preferably 28 to 33 wt%, based on the total weight of the solution including the polymer and the water) which has a gel formation temperature in the range of 25 to 41 °C, more preferably 30 to 41 °C, and even more preferably of 32 to 39 °C. Preferably, the temperature-responsive polymer exhibits a lower critical solution temperature (LCST) in water within the above temperature ranges, such that gel formation will occur when the temperature exceeds the LCST.

The temperature responsive behavior of polymer solutions can be investigated e.g. by rheological experiments. A polymer solution in water (e.g. at a concentration within the range of 5 to 50 wt%, preferably 10 to 40 wt%, more preferably of 25 to 35 wt% and most preferably 28 to 33 wt%, based on the total weight of the solution including the polymer and the water) can be transferred to a rheometer and analyzed by oscillatory shear experiments at a constant frequency, e.g., 0.5-1.0 Hz. A plate-plate geometry can be used. The loss modulus (G"), which reflects the viscous properties of the material, and the storage modulus (G'), which represents the elastic properties of the material, are recorded over time.

The rheological properties are then investigated as a function of the temperature of the solution. At the starting temperature, typically e.g. 20 °C, G" values are higher than G' values and the material behaves like a viscous liquid. The gel point is defined as the temperature where, during the increase of temperature, G' exceeds G" (i.e., the cross-over point of G' and G" in the rheogram). At this point the elastic properties become more dominant than the viscous properties; a gel is formed. For a thermoresponsive polymer solution heating above a defined temperature is accompanied with a strong increase of G'. As described above the gel point is reached when G' exceeds G".

As will be understood by the skilled person, the temperature responsive characteristics of the temperature responsive polymer A and/ or B are provided by the polymer structure (i.e. by the type(s) of repeating units) contained therein. Known temperature responsive polymer structures suitable as a polymer structure of the temperature responsive polymer A and/or B include the poly(N-isopropylacrylamide) structure, and in particular block copolymer structures comprising one or more blocks of a hydrophilic polymer and one or more blocks of a hydrophobic polymer.

A very preferred polymer structure to provide the temperature-responsive polymer A or the temperature responsive polymer B, and preferably for both polymer A and B, is a PEG (polyethylene glycol) /PPG (polypropylene glycol) block copolymer structure comprising one or more poly(ethylene glycol) blocks as blocks of a hydrophilic polymer, and one or more poly(propylene glycol) blocks as blocks of a hydrophobic polymer. Particularly preferred is a PEG/PPG block copolymer structure wherein the repeating units consist of one or more poly(ethylene glycol) blocks as blocks of a hydrophilic polymer, and one or more poly(propylene glycol) blocks as blocks of a hydrophobic polymer. Examples are a poloxamer polymer or a poloxamine polymer as a polymer structure. It will be understood that these PEG/PPG block copolymer structures would be modified as discussed above to carry the diene groups or the dienophile groups, respectively.

If only one of the polymer A and B is a temperature-responsive polymer showing gel formation in water, the polymer structure of the other polymer may be provided e.g. by a hydrophilic polymer such as poly(vinyl alcohol) or poly(ethylene glycol), including modified versions of these polymers having a branched structure.

If polymer A or polymer B, preferably both, have a block copolymer structure comprising one or more poly(ethylene glycol) blocks and one or more poly(propylene glycol) blocks, it is preferred that the block-copolymer structure comprises two or more poly(ethylene glycol) blocks. it is also preferred that the number of poly(ethylene glycol) blocks is equal to or larger than the number of poly(propylene glycol) blocks.

The ratio of polymerized ethylene glycol units to the sum of polymerized ethylene glycol and propylene glycol units in the brock copolymer comprising one or more poly(ethylene glycol) blocks and one or more poly(propylene glycol blocks is preferably in the range of 50 to 90 mol%, more preferably 60 to 80 mol%, and even more preferably 65 to 75 mol%.

As regards the arrangement of the blocks in the block copolymer comprising one or more poly(ethylene glycol) blocks and one or more poly(propylene glycol) blocks, the block-copolymer structure may be a linear polymer structure, such as a poloxamer, or a branched structure, such as a poloxamine. For the linear structure, it is preferred that the polymer A carries at least two, more preferably two, diene groups bound to each polymer chain terminus, and polymer B carries at least two, more preferably two, dienophile groups bound to each polymer chain terminus. As noted above, the diene groups and/or the dienophile groups may be bound to each polymer chain terminus via a branched linker moiety.

However, it is preferred in line with the above that the block copolymer comprising one or more poly(ethylene glycol) blocks and one or more poly(propylene glycol) blocks has a branched structure, more preferably a branched structure wherein each branch of the polymer comprises at least one poly(ethylene glycol) block and at least poly(propylene glycol) blocks. A strongly preferred exemplary branched structure is a branched poly(ethylene glycol) - poly(propylene glycol) block copolymer structure wherein n block copolymer chains, each formed by a poly(propylene glycol) block and a poly(ethylene glycol) block, extend from a n-valent branching moiety, and wherein n is an integer of 3 to 8, preferably 4 to 8, and more preferably 4. Such a branching moiety may be provided, e.g., by a polyvalent alcohol such as pentaerythrit, or by a an amine, diamine or higher valent amine, such as ethylenediamine. In line with the above, particularly preferred branched block copolymers comprising one or more poly(ethylene glycol) blocks and one or more poly(propylene glycol) blocks to provide the polymer structure of polymer A or polymer B, preferably both, are poloxamines.

In line with the above, it is preferred for the branched block copolymer structure comprising one or more poly(ethylene glycol) blocks and one or more poly(propylene glycol) blocks which may provide the polymer structure of polymer A that the polymer structure carries one or more, e.g. one or two, diene groups bound to the termini of the polymer chains of the branched structure, most preferably to the terminus of each polymer chain of the branched structure. Similarly, it is preferred for the branched block copolymer structure comprising one or more poly(ethylene glycol) blocks and one or more poly(propylene glycol) blocks which may provide the polymer structure of polymer B that the polymer structure carries one or more, e.g. one or two, dienophile groups bound to the termini of the polymer chains of the branched structure, most preferably to the terminus of each polymer chain of the branched structure.

Thus, it will be further apparent that it is also preferred in the context of the invention that
(i) polymer A is a poiymer with a branched block copolymer structure comprising one or more poly(ethylene glycol) blocks and one or more poly(propylene glycol) blocks which carries a total of 3 to 12, more preferably 4 to 8, diene groups per molecule,
   wherein one or two of the diene groups are bound to the terminus of each polymer chain of polymer A as pendant reactive groups, and the diene groups are selected from a furane moiety, a cyclopentadiene moiety and a cyclohexadiene moiety, and are more preferably furane moieties, and
(ii) polymer B is a polymer with a branched block copolymer structure comprising one or more poly(ethylene glycol) blocks and one or more poly(propylene glycol) blocks which carries a total of 3 to 12, more preferably 4 to 8, dienophile groups per molecule,
   wherein one or two of the dienophile groups are bound to the terminus of each polymer chain of polymer B as pendant reactive groups, and the dienophile groups are selected from a maleic anhydride moiety, a maleimide moiety, and a 1,4-quinone moiety, and are more preferably maleimide moieties.

Also for this preferred embodiment, it is further preferred that each of polymer A and polymer B has a branched poly(ethylene glycol) - poly(propylene glycol) block copolymer structure wherein n block copolymer chains, each formed by a poly(propylene glycol) block and a poly(ethylene glycol) block, extend from a n-valent branching moiety, and wherein n is an integer of 3 to 8, preferably 4 to 8, and is more preferably 4.

In accordance with a particularly preferred embodiment of the composition in accordance with the invention,
(i) polymer A is a poloxamine polymer wherein 4 polymer chains, each formed by a poly(ethylene glycol) block and a poly(propylene glycol) block, extend from a central 4-valent ethylene diamine moiety, and polymer A carries a total of 4 to 8 diene groups per molecule, wherein one or two of the diene groups are bound to the terminus of each polymer chain of polymer A as pendant reactive groups, and the diene groups are selected from a furane moiety, a cyclopentadiene moiety and a cyclohexadiene moiety, and are more preferably furane moieties, and
(ii) polymer B is a poloxamine polymer wherein 4 polymer chains, each formed by a poly(ethylene glycol) block and a poly(pronylene glycol) block, extend from a central 4-valent ethylene diamine moiety, and polymer B carries a total of 4 to 8 dienophile groups per molecule,
wherein one or two of dienophile groups are bound to the terminus of each polymer chain of polymer B as pendant reactive groups, and the dienophile groups are selected from a maleic anhydride moiety, a maleimide moiety, and a 1,4-quinone moiety, and are more preferably maleimide moieties.

Generally, in the context of the present invention, polymer A may comprise a mixture of two or more, preferably two types of polymer molecules with different numbers of diene groups; and/or polymer B may comprises a mixture of two or more, preferably two types of polymer molecules with different numbers of dienophile groups. It will be understood that the above preferred constitutions disclosed for polymer A and/or polymer B equally apply for polymer A and/or polymer B in such mixtures.

Each of polymer A and polymer B preferably has a number average molecular weight in the range of 500 to 45000 g/mol, more preferably in the range of 1000 to 30000 g/mol, and even more preferably in the range of 2000 to 20000 g/mol. The number average molecular weight can be determined, e.g. via gel permeation chromatography.

As noted above, the present invention provides compositions, preferably pharmaceutical compositions, comprising a plurality of molecules of a polymer A and a plurality of molecules of a polymer B as discussed above. In these compositions, polymer A and polymer B are contained in combination, typically in the form of a mixture.

A preferred additional component of these compositions is a liquid carrier comprising water, in which case polymer A and polymer B are dissolved or dispersed, preferably dissolved, in the liquid carrier comprising water to form an aqueous solution or dispersion. As optional further solvents that can be contained in the liquid carrier, hydrophilic liquid components such as ethylene glycol, polyethylene glycol, propylene glycol or glycerol can be mentioned. Preferably, the liquid carrier comprising water contains water in an amount of 80 vol% or more, more preferably 90 vol% or more, based on the total volume of solvents in the carrier as 100 %. Most preferably, the liquid carrier comprising water contains the water as the only solvent. In view of the fact that the compositions of the present invention are preferably pharmaceutical compositions, the liquid carrier is typically a pharmaceutically acceptable carrier consisting of pharmaceutically acceptable components. Optional further components that may be contained in the liquid carrier include e.g. one or more of a buffer, a pharmaceutically acceptable salt, and other conventional pharmaceutical excipients. For example, the liquid carrier may be a phosphate buffered aqueous saline solution with a pH of 7.4, or an acetate buffered aqueous saline solution with a pH of 5.0.

In another preferred embodiment, the composition in accordance with the invention is a pharmaceutical composition which comprises the polymers A and B as discussed above in combination with a therapeutically active agent. Typically, the polymer A, the polymer B and the therapeutically active agent are present as a mixture. In this embodiment, it is preferred that polymer A and polymer B are dissolved or dispersed, preferably dissolved, in a liquid carrier comprising water to form an aqueous solution or dispersion, and that the therapeutically active agent is also dissolved or dispersed in the liquid carrier. As noted above, as optional further solvents that can be contained in the liquid carrier, hydrophilic liquid components such as ethylene glycol, polyethylene glycol, propylene glycol or glycerol can be mentioned. Preferably, the liquid carrier comprising water contains water in an amount of 80 vol% or more, more preferably 90 vol% or more, based on the total volume of solvents in the carrier as 100 %. Most preferably, the liquid carrier contains water as the only solvent. Optional further components of the liquid carrier include dissolved therein one or more of a buffer, a pharmaceutically acceptable salt, and other conventional pharmaceutical excipients. For example, the liquid carrier may be a phosphate buffered aqueous saline solution with a pH of 7.4, or an acetate buffered aqueous saline solution with a pH of 5.0.

As will be understood by the skilled person, the term "therapeutically active agent" encompasses agents which are suitable to treat or prevent a disorder or disease, especially in a mammalian subject, and in particular in a human subject. As used herein, the term "therapeutically active agent" encompasses agents which prevent the disorder or disease from affecting the subject, e.g. vaccines.

The therapeutically active agent preferably has a molecular weight of at least 100 Da.

As one example of a therapeutically active agent, particles of an active agent with limited solubility in water (at 20 °C), e.g. sparing soluble, slightly soluble, very slightly soluble or practically insoluble drugs.

Preferably, the therapeutically active agent is selected from an antibody, a peptide, a protein, a nucleic acid, an aptamer and an antigen for vaccination, or from combinations thereof, more preferably from an antibody, a peptide, a protein, and an antigen for vaccination, and is even more preferably an antibody.

Where the composition comprises a liquid carrier as discussed above, the total concentration of the polymers A and B in the liquid carrier is preferably within the range of 5 to 50 wt%, more preferably 10 to 40 wt%, even more preferably of 15 to 35 wt%, based on the total weight of the polymers and the liquid carrier.

As will be understood by the skilled reader, the relative concentration of the polymer A and the polymer B in the composition is preferably, adjusted taking into account the structure of polymer A and B such that the ratio of the total number of diene groups to the total number of dienophile groups provided in the composition ranges from 0.8/1.2 to 1.2/0.8, more preferably from 0.9/1.1 to 1.1/0.9.

As further aspects related to the compositions in accordance with the invention, the invention provides the composition in accordance with the invention, preferably the composition comprising a therapeutically active agent, for use in therapy. It also provides the composition in accordance with the invention for use in the therapeutic treatment of a patient via in-situ formation of a hydrogel in the patient's body following the administration of the composition. It will be understood that the composition of the invention also allows a method of treatment to be carried out which comprising the step of administering the composition in accordance with the invention to a patient in need thereof.

The composition, and in particular the pharmaceutical composition, in accordance with the invention wherein polymer A and polymer B are dissolved or dispersed in a liquid carrier comprising water can be conveniently provided by a process comprising the steps of
obtaining the polymer A and the polymer B, and dissolving or dispersing the polymer A and the polymer B in a common liquid carrier comprising water to provide a solution or dispersion comprising a mixture of polymer A and polymer B; or
obtaining the polymer A and the polymer B, dissolving or dispersing the polymer A and the polymer B in separate liquid carriers comprising water to provide a solution or dispersion comprising polymer A and a solution or dispersion comprising polymer B and mixing these solutions or dispersions to provide a solution or dispersion comprising a mixture of polymer A and polymer B.

If the composition is a pharmaceutical composition comprising a therapeutically active agent, the pharmaceutically active agent, the method may further comprise
(i) a step of adding the therapeutically active agent to polymer A, wherein the therapeutically active agent may be added prior to or after dissolving or dispersing polymer A, or
(ii) a step of adding the therapeutically active agent to polymer B, wherein the "therapeutically active agent may be added prior to or after dissolving or dispersing polymer B, or
a step of adding the "therapeutically active agent to the solution or dispersion comprising a mixture of polymer A and polymer B.

in accordance with a further aspect, the invention provides a hydrogel which is obtainable via the gelation of a composition in accordance with the invention as discussed above, said gelation involving the thermal gelation of the composition comprising a plurality of molecules of a polymer A and a plurality of molecules of a polymer B, and the Diels-Alder reaction of the diene reactive groups of polymer A with the dienophile reactive groups of polymer B. As will be understood by the skilled person, the thermal gelation refers to the physical gelation of the temperature responsive polymer(s) upon being exposed to an increase in temperature, which does not give rise to a formation of chemical bonds between the polymer molecules. However, chemical bonds are formed during the Diels-Alder reaction.

Furthermore, it will be understood that the explanation provided above with regard to the compositions of the invention and the polymers A and B, including the explanation regarding preferred embodiments thereof, also applies with respect to the hydrogel of the invention and its formation.

Thus, in a preferred embodiment wherein the hydrogel of the invention is obtainable from a pharmaceutical composition in accordance with the invention comprising a therapeutically active agent, the hydrogel will contain the therapeutically active agent incorporated within the hydrogel polymer network. Such a hydrogel can be conveniently used as a sustained release formulation for the therapeutically active agent. It will be understood that the gelation of a pharmaceutical composition in accordance with the invention can be used to provide a pharmaceutical hydrogel.

The hydrogel in accordance with the invention, in particular the hydrogel obtainable via gelation of the pharmaceutical composition in accordance with the invention may be prepared *in vitro,* but may be provided directly inside the body of a subject (i.e. *in situ*), preferably a mammalian and in particular a human subject.

For the *in vitro* preparation, the components of the composition can be mixed in a suitable order to provide the composition in accordance with the invention, and then the composition is subjected to an increase in temperature. Typically, the mixing occurs at a temperature below 25 °C, e.g. around room temperature of 20 °C or at refrigerator temperatures in the range of 2 to 8 °C. The increase in temperature does not need to be drastic, and the increased temperature at which the gelation occurs is preferably at or above 25 °C, more preferably at or above 30 °C, and even more preferably at or above 32 °C. For the in vitro preparation of a hydrogel in accordance with the invention, the composition can be filled into a container having a predefined shape which is maintained after gelation. The hydrogel prepared in vitro can be administered to a subject by suitable means and pathways.

For the provision of a hydrogel directly inside the body of a subject, it is preferable to provide the composition in accordance with the invention wherein the polymer A and the polymer B, and preferably the therapeutic agent, are dissolved or dispersed in the pharmaceutical acceptable liquid carrier comprising water, and to administer the composition to the subject, e.g. by injection. It is an advantage of the compositions of the present invention that the Diels-Alder reaction proceeds at a low reaction rate e.g. compared to Michael-type crosslinking reactions that have been used for the provisions of a hydrogel. As shown in herein, in the absence of an external stimulus which triggers the thermogelation of the thermo-responsive polymer(s), polymer A and polymer B may coexist in a composition without significant gel formation via Diels-Alder formation over extended periods of time (e.g. more than 30 minutes, preferably more than 1 hour). By contrast, macromonomers functionalized to undergo a Michael addition have been reported to form a gel via crosslinking of the macromonomers over periods of about 100 s in neutral solutions (cf. Pritchard, C.D. et al., Biomaterials 2011, 32, 587-597). Thus, the compositions in accordance with the invention can be conveniently prepared in vitro, and can be administered to a subject without the application being impeded by the premature formation of a gel prior to administration. On the other hand, once it is subjected to the body temperature of the subject, the thermal gelation of the composition occurs rapidly so that e.g. a co-administered therapeutically active agent is quickly incorporated in the hydrogel and is kept from being distributed inside a patient's body while the Diels-Alder reaction leads to a further stabilization of the gel. Nevertheless, if desired, it is also possible to allow the formation of a hydrogel to occur directly inside the body of a patient by administering polymer A and polymer B separately and concurrently to the same site, or separately and subsequently to the same site. The therapeutic agent which is preferably present may be administered in this case e.g. together with polymer A, together with polymer B, or separately to the same site.

The formation of the hydrogel inside the body of the subject is caused by the body temperature of the subject, allowing both the thermal gelation and the Diels-Alder reaction to proceed.

Thus, the present invention also provides a method for the preparation of a hydrogel either *in vitro* or directly in the body of a subject, which comprises a step of preparing a composition in accordance with the invention wherein the polymer A and the polymer B, and preferably the therapeutic agent, are dissolved or dispersed in the liquid carrier comprising water, preferably the pharmaceutically acceptable liquid carrier comprising water, and a step of increasing the temperature of the polymer composition to effect the gelation of a polymer composition, said gelation involving the thermal gelation of the composition comprising a plurality of molecules of a polymer A and a plurality of molecules of a polymer B and the Diels-Alder reaction of the diene reactive groups of polymer A with the dienophile reactive groups of polymer B.

The hydrogel in accordance with the invention can be conveniently prepared. It allows the incorporation of a therapeutically active agent and its controlled released from the hydrogel over an extended period of time within a patient's body. The release rate of the active agent can be controlled by tuning the properties of the hydrogel, e.g. by mixing varying ratios of polymers A and B with different numbers of reactive groups, and/or with different degrees of branching. Thus, the stability or durability, and, as a consequence, the duration of the release can be varied. Furthermore, it is a relevant aspect of the invention that the hydrogel may be degraded in the body of the patient due to occurrence of retro-Diels-Alder (rDA) reactions cleaving the bonds formed by the Diels-Alder reaction.

As further aspects related to the hydrogel in accordance with the invention, the invention provides the hydrogel in accordance with the invention, preferably the hydrogel comprising a therapeutically active agent, for use in therapy. It also provides the hydrogel in accordance with the invention for use in the therapeutic treatment of a patient involving the *in-situ* formation of the hydrogel in the patient's body.

In still a further aspect, the invention provides a kit of parts comprising
a plurality of molecules of a polymer A and a plurality of molecules of a polymer B, wherein
polymer A carries two or more diene groups bound to the polymer structure as pendant reactive groups for a Diels-Alder reaction with polymer B;
polymer B carries two or more dienophile groups bound to the polymer structure as pendant reactive groups for a Diels-Alder reaction with polymer A;
and wherein at least one of polymer A and polymer B is a temperature-responsive polymer.

It will be understood that in such a kit, the polymers A and B are provided in association, but are not in physical contact with each other. For example, the polymer A and the polymer B can be provided in a set of containers provided together, or in separate compartments of a single container. Thus, the kit is suitable for providing a composition in accordance with the invention.

Furthermore, it will be understood that the explanation provided above with regard to the compositions of the invention and the polymers A and B, including the explanation regarding preferred embodiments thereof, also applies with respect to the kit of parts of the invention.

For example, also for the kit of parts it is preferred that it further comprises the liquid carrier comprising water, more preferably the pharmaceutically acceptable liquid carrier comprising water, as discussed above for the composition in accordance with the invention. For example, at least one of polymer A and polymer B, preferably both, may be dissolved or dispersed in the liquid carrier, or the liquid carrier may be provided separately from polymers A and B, e.g. in a separate container, or in a separate compartment in the same container.

Moreover, also for the kit of parts it is preferred that it further comprises the therapeutically active agent as discussed above for the composition in accordance with the invention. For example, at least one of polymer A and polymer B may be mixed with the therapeutically active agent, or the therapeutically active agent may be provided separately from polymers A and B, e.g. in a separate container, or in a separate compartment in the same container.

Thus, in a preferred embodiment, the kit of parts comprises
a plurality of molecules of a polymer A as discussed above and a plurality of molecules of a polymer B as discussed above, wherein
polymer A carries two or more diene groups bound to the polymer structure as pendant reactive groups for a Diels-Alder reaction with polymer B;
polymer B carries two or more dienophile groups bound to the polymer structure as pendant reactive groups for a Diels-Alder reaction with polymer A;
and wherein at least one of polymer A and polymer B is a temperature-responsive polymer; the pharmaceutically acceptable liquid carrier comprising water as discussed above for the composition in accordance with the invention, and
the therapeutically active agent as discussed above for the composition in accordance with the invention.

Important aspects of the present inventions are summarized in the following items, which reflect the general aspects of the present invention as well as preferred embodiments thereof.
1. A composition comprising a plurality of molecules of a polymer A and a plurality of molecules of a polymer B, wherein
   polymer A carries two or more diene groups bound to the polymer structure as pendant reactive groups for a Diels-Alder reaction with polymer B;
   polymer B carries two or more dienophile groups bound to the polymer structure as pendant reactive groups for a Diels-Alder reaction with polymer A;
   and wherein at least one of polymer A and polymer B is a temperature-responsive polymer.
2. The composition according to item 1, wherein at least one of polymer A and polymer B carries three or more of the reactive groups for a Diels-Alder reaction.
3. The composition according to item 1 or 2, wherein polymer A carries three or more, more preferably four or more diene groups bound to the polymer.
4. The composition according to any of items 1 to 3, wherein polymer B carries three or more, more preferably four or more dienophile groups bound to the polymer.
5. The composition according to any of items 1 to 4, wherein the diene groups bound to polymer A are selected from a furane moiety, a cyclopentadiene moiety and a cyclohexadiene moiety.
6. The composition according to any of items 1 to 4, wherein the diene groups bound to polymer A are furane moieties.
7. The composition according to any of items 1 to 6, wherein the dienophile groups bound to polymer B are selected from a maleic anhydride moiety, a maleimide moiety, and a 1,4-quinone moiety.
8. The composition according to any of items 1 to 6, wherein the dienophile groups bound to polymer B are maleimide moieties.
9. The composition according to any of items 1 to 8, wherein polymer A is a polymer carrying one or two diene groups bound to each polymer chain terminus.
10. The composition according to any of items 1 to 8, wherein polymer A has a branched polymer structure carrying one or two diene groups bound to each polymer chain terminus.
11. The composition according to any of items 1 to 10, wherein polymer B is a polymer carrying one or two dienophil groups bound to each polymer chain terminus.
12. The composition according to any of items 1 to 10, wherein polymer B has a branched polymer structure carrying one or two diene groups bound to each polymer chain terminus.
13. The composition according to any of items 1 to 12, wherein the temperature-responsive polymer has a gel formation temperature in water in the range of 25 to 41 °C, more preferably 30 to 41 °C, and even more preferably of 32 to 39 °C.
14. The composition according to any of items 1 to 13, wherein polymer A and B are both a temperature responsive polymer.
15. The composition according to any of items 1 to 14, wherein the temperature-responsive polymer has a block copolymer structure comprising one or more blocks of a hydrophilic polymer and one or more blocks of a hydrophobic polymer.
16. The composition according to item 15, wherein the temperature-responsive polymer has a block copolymer structure comprising one or more poly(ethylene glycol) blocks as blocks of a hydrophilic polymer, and one or more poly(propylene glycol) blocks as blocks of a hydrophobic polymer.
17. The composition according to item 17, wherein the temperature-responsive polymer comprises two or more poly(ethylene glycol) blocks.
18. A composition comprising a plurality of molecules of a polymer A and a plurality of molecules of a polymer B, wherein
   polymer A has a block copolymer structure comprising one or more poly(ethylene glycol) blocks and one or more poly(propylene glycol) blocks, and carries two or more diene groups bound to the polymer structure as pendant reactive groups for a Diels-Alder reaction with polymer B;
   polymer B has a block copolymer structure comprising one or more poly(ethylene glycol) blocks and one or more poly(propylene glycol) blocks, and carries two or more dienophile groups bound to the polymer structure as pendant reactive groups for a Diels-Alter reaction with polymer A.
19. The composition according to item 18, wherein polymer A and polymer B each comprises two or more poly(ethylene glycol) blocks.
20. The composition according to item 18 or 19, wherein at least one of polymer A and polymer B carries three or more of the reactive groups for a Diels-Alder reaction.
21. The composition according to any of items 18 to 20 wherein polymer A carries three or more, more preferably four or more diene groups bound to the polymer.
22. The composition according to any of items 18 to 21, wherein polymer B carries three or more, more preferably four or more dienophile groups bound to the polymer.
23. The composition according to any of items 18 to 22, wherein the diene groups bound to polymer A are selected from a furane moiety, a cyclopentadiene moiety and a cyclohexadiene moiety.
24. The composition according to any of items 18 to 22, wherein the diene groups bound to polymer A are furane moieties.
25. The composition according to any of items 18 to 24, wherein the dienophile groups bound to polymer B are selected from a maleic anhydride moiety, a maleimide moiety, and a 1,4-quinone moiety.
26. The composition according to any of items 18 to 24, wherein the dienophile groups bound to polymer B are maleimide moieties.
27. The composition according to any of items 18 to 26, wherein polymer A is a polymer carrying one or two diene groups bound to each polymer chain terminus.
28. The composition according to any of items 18 to 26, wherein polymer A has a branched polymer structure carrying one or two diene groups bound to each polymer chain terminus.
29. The composition according to any of items 18 to 28, wherein polymer B is a polymer carrying one or two dienophil groups bound to each polymer chain terminus.
30. The composition according to any of items 18 to 28, wherein polymer B has a branched polymer structure carrying one or two diene groups bound to each polymer chain terminus.
31. The composition according to any of items 16 to 30, wherein the number of poly(ethylene glycol) blocks is equal to or larger than the number of poly(propylene glycol) blocks in the block copolymer comprising one or more poly(ethylene glycol) blocks and one or more poly(propylene glycol) blocks.
32. The composition according to any of items 16 to 31, wherein the ratio of polymerized ethylene glycol units to the sum of polymerized ethylene glycol and propylene glycol units in the block copolymer comprising one or more poly(ethylene glycol) blocks and one or more poly(propylene glycol) blocks is in the range of 50 to 90 mol%, more preferably 60 to 80 mol%, and even more preferably 65 to 75 mol%.
33. The composition according to any of items 16 to 32, wherein the each of polymer A and polymer B has a branched poly(ethylene glycol) - poly(propylene glycol) block copolymer structure wherein n block copolymer chains, each formed by a poly(propylene glycol) block and a poly(ethylene glycol) block, extend from a n-valent branching moiety, and wherein n is an integer of 3 to 8.
34. The composition according to item 33, wherein n is 4 to 8, more preferably 4.
35. The composition according to item 33 or 34, wherein each of polymer A and polymer B has a poloxamine polymer structure.
36. The composition according to any of items 16 to 27, 29 and 31 to 33, wherein each of polymer A and B has a poloxamer polymer structure, and wherein polymer A carries at least two diene groups bound to each polymer chain terminus, and polymer B carries at least two dienophil groups bound to each polymer chain terminus.
37. The composition according to item 36 wherein the at least two diene groups and the at least two dienophile groups, respectively, are bound to each polymer chain terminus via a branched linker moiety.
38. The composition according to any of items 1 to 37, wherein:
   (i) polymer A comprises a mixture of two or more, preferably two types of polymer molecules with different numbers of diene groups; and/or
   (ii) polymer B comprises a mixture of two or more, preferably two types of polymer molecules with different numbers of dienophile groups.
39. The composition according to any of items 1 to 38, wherein the number average molecular weight of each of polymer A and of polymer B is in the range of 500 to 45000 g/mol, more preferably in the range of 1000 to 30000 g/mol, and even more preferably in the range of 2000 to 20000 g/mol.
40. The composition according to any of items 1 to 39, wherein polymer A and polymer B are dissolved or dispersed in a liquid carrier comprising water.
41. The composition according to item 40, which is a pharmaceutical composition and wherein the liquid carrier is a pharmaceutically acceptable liquid carrier comprising water.
42. The composition according to any of items 1 to 39 which is a pharmaceutical composition comprising one or more therapeutically active agents.
43. The composition according to item 42, wherein polymer A, polymer B and the one or more therapeutically active agents are dissolved or dispersed in a pharmaceutically acceptable liquid carrier comprising water.
44. The composition according to item 42 or 43, wherein the therapeutically active agent has a molecular weight of at least 100 Da.
45. The composition according to any of items 42 to 44, wherein the therapeutically active agent is selected from an antibody, a peptide, a protein, a nucleic acid, an aptamer and an antigen for vaccination, or from combinations thereof, more preferably from an antibody, a peptide, a protein, and an antigen for vaccination.
46. The composition according to any of items 42 to 44, wherein the therapeutically active agent is a peptide or a protein.
47. A hydrogel which is obtainable via the gelation of a composition in accordance with any of items 1 to 46, said gelation involving the thermal gelation of the composition comprising a plurality of molecules of a polymer A and a plurality of molecules of a polymer B, and the Diels-Alder reaction of the diene reactive groups of polymer A with the dienophile reactive groups of polymer B.
48. A kit of parts comprising
   a plurality of molecules of a polymer. A and a plurality of molecules of a polymer B, which polymers are defined as in any one of items 1 to 39.
49. The kit of parts in accordance with item 48, wherein the polymer A and the polymer B are contained in separate compartments of a common container.
50. The kit of parts in accordance with item 48 or 49, which further comprises a pharmaceutically acceptable liquid carrier comprising water, and wherein the liquid carrier is separate from polymer A and polymer B, or wherein polymer A and polymer B are separately dissolved or dispersed in the liquid carrier.
51. The kit of parts in accordance with any of items 48 to 50, which further comprises one or more therapeutically active agents.
52. A method of treatment comprising the step of administering a composition in accordance with any of items 1 to 46 to a patient.
53. The composition in accordance with any of items 1 to 46 for use in therapy.
54. A composition in accordance with any of items 1 to 47 for use in the therapeutic treatment of a patient via in-situ formation of a hydrogel in the patient's body following the administration of the composition.
55. A method for the preparation of a composition in accordance with any of items 40 to 46 wherein polymer A and polymer. B are dissolved or dispersed in a liquid carrier comprising water, said method comprising the steps of:
   obtaining the polymer A and the polymer B, and dissolving or dispersing the polymer A and the polymer B in a common liquid carrier comprising water to provide a solution or dispersion comprising a mixture of polymer A and polymer B; or
   obtaining the polymer A and the polymer B, dissolving or dispersing the polymer A and the polymer B in separate liquid carriers comprising water to provide a solution or dispersion comprising polymer A and a solution or dispersion comprising polymer B and mixing these solutions or dispersions to provide a solution or dispersion comprising a mixture of polymer A and polymer B.
56. The method of item 55, which further comprises (i) a step of adding a therapeutically active agent to polymer A, wherein the therapeutically active agent may be added prior to or after dissolving or dispersing polymer A, and/or (ii) a step of adding a therapeutically active agent to polymer B, wherein the therapeutically active agent may be added prior to or after dissolving or dispersing polymer B.
57. A method for the preparation of a hydrogel, said method comprising a step of preparing a composition in accordance with the method of item 55 or 56, and a step of increasing the temperature of the polymer composition to effect the gelation of a polymer composition, said gelation involving the thermal gelation of the composition comprising a plurality of molecules of a polymer A and a plurality of molecules of a polymer B and the Diels-Alder reaction of the diene reactive groups of polymer A with the dienophile reactive groups of polymer B.

### Examples

### Materials and Methods

### Materials

Boc-6-aminohexanoic acid and Fmoc-Lys(Boc)-OH were purchased from Bachem (Weil am Rhein, Germany). 3-(4,5-dimethylthiazol-2-yl)-2,5-diphenyltetrazolium bromide (MTT) was obtained from PanReac AppliChem (Darmstadt, Germany). Poloxamine (Tetronic^{®} 1107) was provided by BASF SE (Ludwigshafen am Rhein, Germany). Deuterated chloroform (CDCl₃), N,N'-dicyclohexylcarbodiimide (DCC), Eagle's minimum essential medium (EMEM), fetal calf serum (FCS), 3-(2-furyl)propanoic acid, N-hydroxysuccinimide (NHS), and N-methoxycarbonylmaleimide were received from Sigma-Aldrich (Taufkirchen, Germany). Mouse fibroblast L-929 cells were a kind gift from the group of Prof. Armin Buschauer (University of Regensburg). All other chemicals were obtained from Merck KGaA (Darmstadt, Germany). Bevacizumab (Avastin^{®}, 25 mg mL⁻¹, Roche Ltd, Basel, Switzerland) was kindly provided by the hospital pharmacy of the University of Regensburg (Germany). Purified water was freshly prepared using a Milli-Q water purification system (Millipore, Schwalbach, Germany).

### ¹H-NMR spectroscopy

¹H-NMR spectra were recorded in CDCl₃ at 295 K using a Bruker Avance 300 spectrometer (Bruker BioSpin GmbH, Rheinstetten, Germany).

### Synthesis of branched polymers with four polymer chains and one functional group at each chain terminus (4arm macromonomers)

Poloxamines were end-functionalized with maleimide or furyl moieties as described for poly(ethylene glycol) (PEG) based macromonomers in Brandl, F. et al., Adv. Eng. Mater. 2007, 9 (12), 1141-1149 or Kirchhof, S. et al., J. Mater. Chem. B 2013, 1 (37), 4855-4864. In brief, poloxamine (Tetronic® 1107) was converted to 4armPoloxamine-NH₂ by means of a Mitsunobu reaction followed by hydrazinolysis. 4armPoloxamine-NH₂ was then reacted with N-methoxycarbonylmaleimide to yield 4armPoloxamine-maleimide. The reaction of 4armPoloxamine-NH2 with 3-(2-furyl)propanoic acid, DCC, and NHS led to 4armPoloxamine-furan. The degree of end-group functionalization was 65-82% for 4armPoloxamine-maleimide and 80-83% for 4armPoloxamine-furan as determined by ¹H-NMR.

### Synthesis of of branched polymers with four polymer chains and two functional group at each chain terminus (8arm macromonomers)

8arm poloxamines were synthesized as described for PEG-based macromonomers in Kirchhof, S. et al., Eur. J. Pharm. Biopharm. 2015, 96, 217-225. Briefly, 4armPoloxamine-NH2 was first reacted with Fmoc-Lys(Boc)-OH, DCC and NHS. The Fmoc-groups were removed and Boc-6-aminohexanoic acid was reacted with the free amino-group via DCC/NHS chemistry. All Boc-groups were then removed and the free amino-groups were functionalized with furyl (8armPoloxamine-furan) or maleimide moieties (8armPoloxamine-maleimide) as described above. The degree of end-group functionalization was 61-71% for 8armPoloxamine-maleimide and 77-85% for 8armPoloxamine-furan as determined by 1H-NMR.

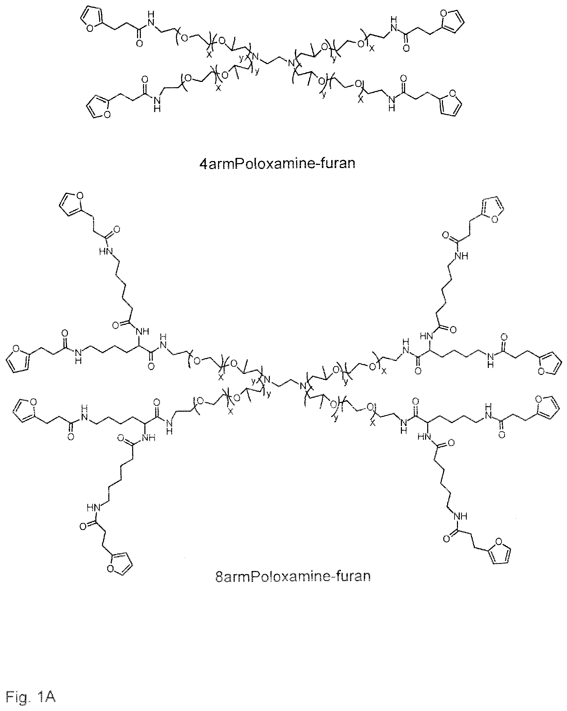

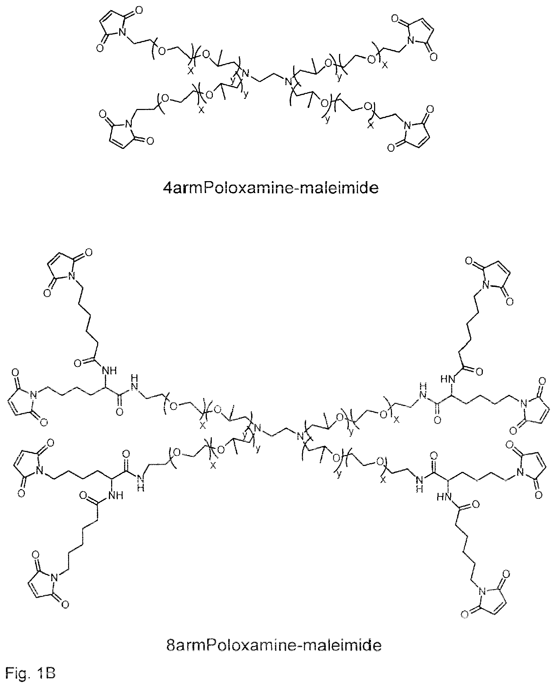

Fig. 1 shows the 4arm and 8arm poloxamines provided with furyl (Fig. 1A) and maleimide (Fig. 1B) groups, respectively. Macromonomers were synthesized from Tetronic^{®} 1107; the x (poly(ethylene glycol, PEG) to y (poly(propylene glycol, PPG) ratio was 70:30. Branching of the linkers carrying two functional groups in the 8arm poloxamines was achieved by introducing lysine and 6-aminohexanoic acid residues.

### Gel formation and rheological properties

For hydrogel preparation, the polymers carrying the diene groups and the polymers carrying the dienophile groups were dissolved in water using a total polymer concentration of 30 wt.%. Various ratios between 8armPoloxamine and 4armPoloxamine were used to prepare hydrogels with different properties. For example, in order to prepare 1000 mg of a hydrogel composed of 20 wt.% 8armPoloxamine and 10 wt.% 4armPoloxamine, i.e., a 20%/10% hydrogel, a total amount of 300 mg polymer was dissolved in 700 mg water. Specifically, for this type of hydrogel 100 mg 8armPoloxamine-maleimide, 100 mg 8armPoloxamine-furan, 50 mg 4armPoloxamine-maleimide, and 50 mg 4armPoloxamine-furan were used. Immediate gel formation could be induced by increasing the temperature to 37 °C.

Rheological characterization was performed on a TA instruments AR 2000 rheometer (TA Instruments, Eschborn, Germany). Oscillatory shear experiments were carried out using a 40 mm parallel plate geometry with a 500 µm gap size at a constant frequency of 0.5 Hz. Polymer solutions were prepared as described above; a sample volume of 750 µL was used. Absolute values of the complex shear modulus (|G*|) and phase angles (δ) were recorded as a function of time. In order to characterize the thermoresponsive behavior of the polymer solutions, heating (20 °C to 37 °C) and cooling (37 °C to 20 °C) steps were included in the procedure. Water evaporation was minimized by using a solvent trap and by pipetting silicone oil around the gap between the plates. However, in order to minimize the influence of drying on the obtained |G*| values, rheological experiments were limited to 100 minutes.

### Swelling and degradation

Swelling and degradation of DA-Poloxamine hydrogels was studied in 50 mM phosphate buffer, pH 7.4 at 37 °C as described in Kirchhof, S. et a!., F. P. Eur. J. Pharm. Biopharm. 2015, 96, 217-225. Different ratios between 8armPoloxamine and 4armPoloxamine were used as described above; a polymer concentration of 30 wt.% was used for all hydrogels.

### Hydrolytic stability of maleimides

The hydrolytic stability of 4armPoloxamine-maleimide and 8armPoloxamine-maleimide was investigated in phosphate buffer, pH 7.4 at 37 °C as described in Gregoritza, M. et al., F. P. J. Mater. Chem. B 2016, 4, 3398-3408.

### Antibody release and analytics

Release experiments with antibodies as an active agent were carried out as described in Gregoritza, M.et al., J. Mater. Chem. B 2016, 4, 3398-3408. However, in this investigation the bevacizumab loading was 6.25 mg mL⁻¹ and the polymer concentration used was 30 wt.%. Due to the high stability of some hydrogels, release experiments were only carried out using 0%/30%, 5%/25%, and 10%/20% hydrogels. In order to investigate a potential negative influence of hydrogel preparation and cross-linking reactions on the stability of bevacizumab, the antibody was analyzed after release. Functional binding to vascular endothelial growth factor (VEGF) was investigated using a Shikari^{®} Q-beva enzyme-linked immunosorbent assay (ELISA) kit (Matriks Biotek, Ankara, Turkey). The amount of binding antibody was compared to a solution containing the same concentration of fresh bevacizumab.

### Statistical analysis

All results are presented as mean ± standard deviation, based on the data obtained from at least *n* = 3 samples. Statistical significance was determined by means of one-way ANOVA, followed by Tukey's *post-hoc* test using GraphPad Prism 6.0 (GraphPad Software Inc., La Jolla, CA, USA). Differences were considered statistically significant at p < 0.05.

### Results

### Results of dual gelation experiments

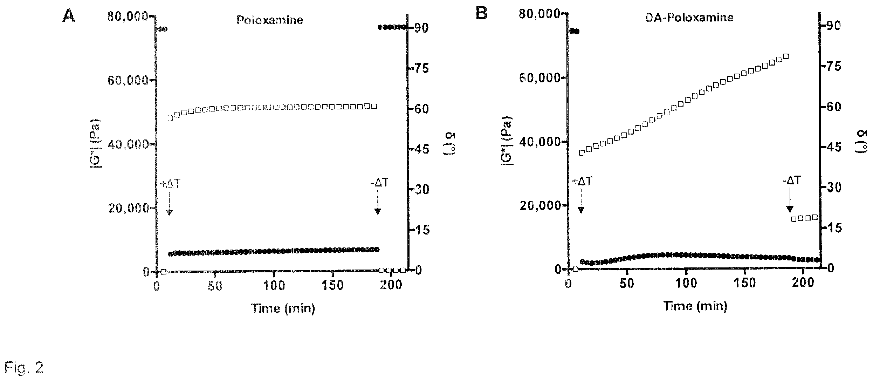

In a first experiment, the rheological properties of a mixture of 4armPoloxamines modified with diene and dienophil groups, respectively ("DA-Poloxamine") were investigated and compared to unmodified commercially available Tetronic^{®} 1107 ("Poloxamine"). For this purpose, 30 wt.% polymer was dissolved in water and analyzed using a three-step temperature program (Fig. 2). Absolute values of the complex shear modulus |G*| and phase angles were recorded over time to monitor gel formation and stiffness. At 20 °C, both Poloxamine and DA-Poloxamine were in the liquid state which was confirmed by |G*| values of 0.7 ± 0.2 Pa for Poloxamine and 0.8 ± 0.4 Pa for DA-Poloxamine. The phase angles (δ) were 90.1 ± 0.2° (Poloxamine) and 88.5 ± 2.4° (DA-Poloxamine) indicating mainly viscous behavior. Upon increasing the temperature to 37 °C (+ΔT), the |G*| values for both sample types immediately increased confirming their thermoresponsive properties. Directly after heating, the |G*| values were 48.6 ± 6.9 kPa for Poloxamine and 36.2 ± 9.8 kPa for DA-Poloxamine; differences were not statistically significant (p < 0.05). At the same time, δ strongly decreased for both samples indicating the formation of a viscoelastic gel. During the following incubation time at 37 °C, the stiffness of Poloxamine gels remained unchanged. In contrast, |G*| values for DA-Poloxamine gels increased to 67.0 ± 13.7 kPa. The increase in stiffness was attributed to covalent cross-linking reactions which occurred while already in the gel state. it could also be observed that the increase in |G*| was mainly governed by the strongly rising storage modulus (G'). The contribution of the loss modulus (G") was much weaker. In a third step, the temperature was lowered to 20 °C (-ΔT). Unmodified Poloxamine gels returned to the liquid state (|G*| = 0.7 ± 0.3 Pa, δ = 90.3 ± 0.2°) whereas DA-Poloxamines remained in the gel state (|G*| = 15.3 ± 3.2 kPa, δ = 3.5 ± 0.3°). Taken together, the experiment confirmed that DA-Poloxamines exhibit thermal gelation and DA cross-linking in a dual gelation process.

Figure 2 shows the rheological behavior of 30 wt.% Poloxamine (Fig. 2A) and DA-Poloxamine (Fig. 2B) solutions in the course of a three-step temperature program. Absolute values of the complex shear modulus (|G*|), represented as white squares, and phase angles (δ), represented as black circles, were recorded as a function of temperature and time at a 0.5 Hz oscillatory frequency. The initial temperature was set to 20 °C. After 10 min, the temperature was increased to 37 °C (+ΔT) and kept constant for 180 min. In the third step, the temperature was again reduced to 20°C (-ΔT). Both polymer solutions displayed thermal gelation, however, only for DA-Poloxamines stiffness increased during incubation and could be retained after cooling. For the sake of clarity, error bars are not shown in the diagram.

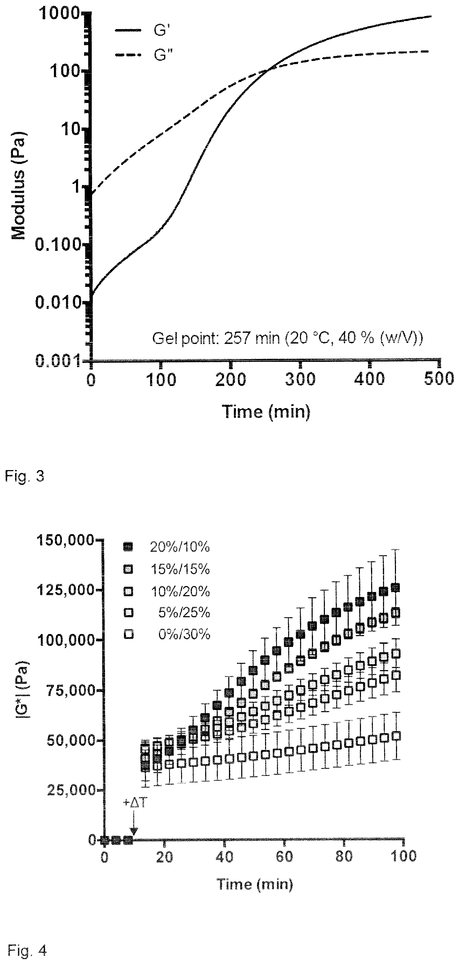

In addition, the gelation of four-armed DA-Poloxamine (40% (w/V)) was monitored at a constant temperature of 20 °C in an oscillatory shear experiment. The rheological measurement was performed using a plate-plate geometry at a constant frequency of 0.5 Hz. The solvent was purified water and a solvent trap with silicone oil was used to minimize evaporation. As shown in Fig. 3, gelation occurred under these conditions due to the Diels-Alder reaction of the diene group-functionalized macromonomers and the dienophil group-functionalized macromonomers after circa 257 minutes.

### Results for gel formation and mechanical properties

Hydrogels with tunable properties were provided by employing different ratios of 4arm and 8arm macromonomers for hydrogel preparation. In this way, hydrogels with different amounts of functional groups and cross-linking densities could be obtained. In the following section, hydrogels composed of both 4armPoloxamines and 8armPoloxamines were analyzed for their thermal gelation properties and gel stiffness. In the nomenclature used to describe the compositions, the first number denotes the amount of 8armPoloxamine in the mixture, the second number refers to the amount of 4armPoloxamine. For example, in order to prepare 1000 mg of a 20%/10% hydrogel, 100 mg 8armPoloxamine-maleimide, 100 mg 8armPoloxamine-furan, 50 mg 4armPoloxamine-maleimide, and 50 mg 4armPoloxamine-furan were dissolved in 700 mg water. The total polymer concentration was 30 wt.% for all hydrogel types.

In the next experiment, the rheological behavior of hydrogels with different 8arm to 4arm ratios was analyzed (Fig. 4). |G*| was recorded at 20 °C for 10 min. Then, the temperature was increased to 37 °C and kept constant for 90 min. initially, all compositions were liquid and did not show significant stiffness; |G*| values ranged between 0.5 and 0.8 Pa. After 10 min at 20 °C the stiffness had slightly increased due to the beginning DA cross-linking, for example to 1.0 ± 0.6 for 0%/30% gels and to 1.7 ± 0.3 Pa for 20%/10% gels. Here, the slow reaction kinetic of the DA reaction is an advantage as faster covalent cross-linking could cause faster gel formation and might prevent administration, e.g., by clogging the needle.

Upon increasing the temperature to 37 °C, stable viscoelastic gels were formed immediately. |G*| values determined directly after increase in temperature are listed in Table 1. For all compositions, a strong increase in stiffness was observed. Interestingly, there was no significant difference between |G*| values directly after the increase in temperature (p < 0.05). Consequently, the strength of the thermally induced gel formation is governed by the backbone and is hardly affected by the number of functional end-groups on the macromonomer. The thermoresponsive properties were comparable for all compositions.

Figure 4 shows the initial thermal gelation and subsequent increase of stiffness for different 8armPoloxamine and 4armPoloxamine mixtures using an overall polymer concentration of 30 wt.%. Absolute values of the complex shear moduli (|G*|) were recorded over time at a 0,5 Hz oscillatory frequency. The initial temperature was set to 20 °C. After 10 min the temperature was increased to 37 °C (+ΔT) and then kept constant for 90 min. All polymer solutions were characterized by an immediate increase of stiffness at 37 °C. The further increase of stiffness was attributed to covalent cross-linking via Diels-Alder reaction and was stronger when more functional end-groups were present in the solution.

In contrast, the increase in stiffness during incubation at 37 °C strongly depended on the 8arm/4arm-ratio used. For 0%/30% hydrogels, a |G*| increase of only 1.4-fold could be observed. However, the |G*| increase was considerably more pronounced for hydrogeis containing a higher concentration of branched macromonomers, e.g., 3.6-fold for 20%/10% hydrogels.

**Table 1: Absolute values of the complex shear modulus (|G*|) determined for hydrogels with different 8armPoloxamine to 4armPoloxamine ratios, measured directly after increase of temperature (0 min) and after incubation (90 min).**

| Hydrogel composition | \|G*\| (0 min) | \|G*\| (90 min) |
|---|---|---|
| 20%/10% | 35.6 ± 10.3 kPa | 126.7 ± 19.0 kPa |
| 15%/15% | 40.6 ± 1.3 kPa | 114.0 ± 2.6 kPa |
| 10%/20% | 45.2 ± 3.7 kPa | 94.0 ± 7.2 kPa |
| 5%/25% | 46.1 ± 3.9 kPa | 83.0 ± 8.5 kPa |
| 0%/20% | 36.2 ± 9.8 kPa | 52.1 ± 11.9 kPa |

### Results for hydrogel swelling and degradation

Swelling and degradation behavior of DA-Poloxamine gel cylinders was analyzed in phosphate buffer, pH 7.4 at 37 °C (Fig. 4A).

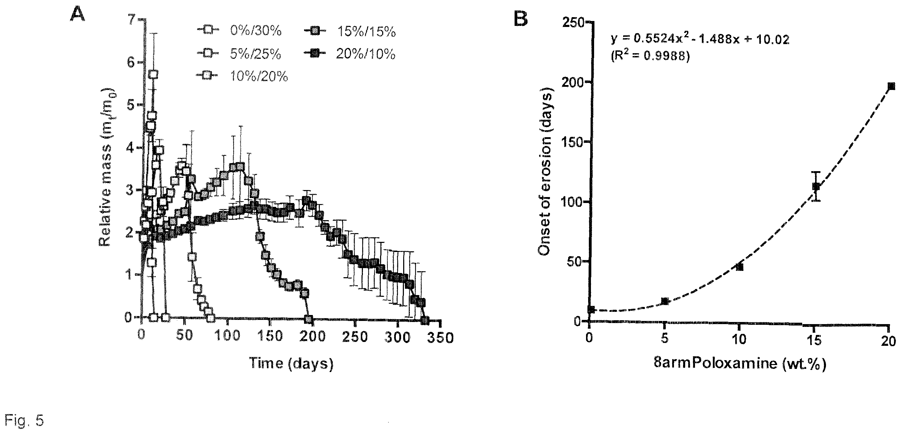

Figure 5 shows the swelling and degradation of DA-Poloxamine hydrogels with different compositions in phosphate buffer, pH 7.4 at 37 °C (Fig. 5A). A higher number of functional end-groups correlated with an increase in stability and a lower degree of swelling. The onset of hydrogel erosion, i.e., beginning decrease of relative hydrogel mass, could be correlated with the concentration of 8armPoloxamine using a second-order polynomial (Fig. 5B).

Similar to hydrogel stiffness, the stability and swelling properties of DA-Poloxamine hydrogel can be controlled by choosing the right composition. For example, hydrogels prepared based on four-armed macromonomers alone (0%/30%) completely dissolved after approximately 14 days. The maximum relative mass increase was 5.7 ± 1.0. In comparison, hydrogels containing the highest amount of branched macromonomers (10%/20%) completely dissolved after 329 days and reached a relative mass of only 2.9 ± 0.3.

In order to select the right composition for a certain application it would be highly desirable to be able to predict hydrogel stability. Therefore, the relationship between onset of hydrogel erosion and 8armPoloxamine content was investigated (Fig. 5B). The onset of erosion was defined as the time-point where the relative hydrogel mass started to decrease. Interestingly, the relation between 8armPoloxamine content and onset erosion can be described using a second-order polynomial (R²=0.9988). This correlation can be used to predict the stability of a specific composition, or to tailor hydrogel stability for a given application.

### Results for controlled antibody release

In order to assess the potential for the delivery of active agents using the hydrogels provided by the compositions of the invention, antibody release from DA-Poloxamine hydrogels was studied. To this end, three hydrogel compositions were loaded with the model antibody bevacizumab (Avastin^{®}) and the *in vitro* release was analyzed in phosphate buffer, pH 7.4 at 37 °C (Fig. 5).

As expected from swelling and degradation experiments, the release rate was influenced by hydrogel composition. The least stable hydrogel (0%/30%) released the antibody after 7 days. When higher ratios of branched macromonomer was used for gel preparation, antibody release could be significantly delayed. Bevacizumab was released over the course of 21 days for 5%/25% hydrogels and 115 days for 10%/20% hydrogels. In all three cases, more than 90% of the antibody that had been loaded could be recovered after release. Interestingly, the release profiles for the three hydrogels types all had a comparable shape, but on different time-scales. Initially, a notably low burst release of about 2 - 6% was observed. After a short lag phase, a rapid antibody release could be observed. The fast release can be explained by an increase of meshwork size resulting from hydrogel swelling. Consequently, antibodies were able to freely diffuse out of the network. The cumulative antibody release in the first phase was 67.9 ± 8.0 (0%/30%), 53.2 ± 4.9 (5%/25%), and 30.3 ± 3.9 (10%/20%). The amount of antibody released in this phase was higher for hydrogels with a lower number of covalent cross-links. In the next phase, antibody release was considerably slower. During this plateau phase, the majority of antibody was trapped within the polymer network. During this phase, single chains were broken over time due to rDA (retro Diels-Alder) reaction and maleimide hydrolysis. Consequently, antibody release was delayed. In case of the most stable gel, this plateau phase spanned over 93 days. In the third phase, a rapid antibody release was observed. The constant breaking of chains led to a gradual increase of average meshwork size until a critical meshwork size had been exceeded. At this point, the previously entrapped antibody was rapidly released.

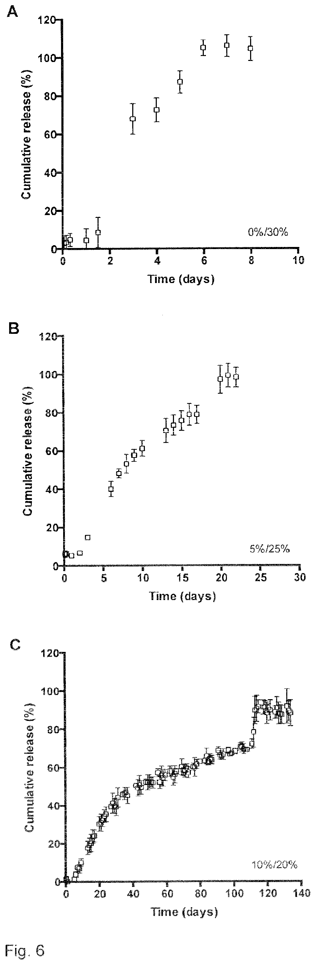

Figure 6 shows the release of bevacizumab from 0%/30%, 5%/25%, and 10%/20% DA-Poloxamine hydrogels in phosphate buffer, pH 7.4 at 37 °C.

A tri-phasic release profile could be beneficial for various applications. One example could be in the treatment of ocular neovascularizations, in which anti-VEGF antibodies are directly administered into the vitreous body. The ocular half-life of antibodies is 7-10 days, so that sustained release systems could be used to provide sufficient antibody concentrations over weeks. However, as VEGF is a key player in maintaining cone photoreceptors and the choroidal vasculature, a constant blockage of VEGF signaling might lead to additional side-effects. The intermittent release profile provided by DA-Poloxamine hydrogels might be an attractive alternative as VEGF-dependent structures can recover from the action of VEGF-neutralizing antibodies during the plateau phase. Another potential application for DA-Poloxamine hydrogels could be in the administration of vaccines. The intermittent release profile could be used to deliver antigens in a pulsed manner following a single administration.

Finally, to assess whether hydrogel preparation and cross-linking could impair protein stability, the binding ability of released bevacizumab was analyzed. To this end, bevacizumab was incorporated into 0%/30% and 5%/25% hydrogels. The hydrogels were then allowed to fully degrade for 7 days and 30 days, respectively. Samples were taken and bevacizumab binding to VEGF was analyzed using ELISA. Fresh bevacizumab in the same concentration as the sample solution was used as a reference. For both hydrogel types, most of the released bevacizumab retained its binding properties (Table 2). The proportion of binding antibody was 87.0 ± 5.6% for 0%/30% hydrogels and 87.1 ± 1.7% for 5%/25% hydrogels. Consequently, preparation, thermal gelation and cross-linking had only a minimal influence on antibody binding.

**Table 2: Analytical investigation of bevacizumab released from DA-Poloxamine hydrogels. Bevacizumab binding to VEGF was quantified using ELISA; results were compared to a solution containing the same concentration of fresh bevacizumab.**

| Hydrogel composition | Time-scale (days) | Binding antibody (%) |
|---|---|---|
| 0%/30% | 7 | 87.0 ± 5.6 |
| 5%/25% | 30 | 87.1 ± 1.7 |

To summarize, it could be demonstrated that the hydrogels provided herein are an outstanding material for the controlled release of therapeutic agents, including complex agents such as antibodies. Time-scales of release can be influenced by hydrogel composition. The general release profile was independent from hydrogel composition and is characterized by three phases and a low burst release. In all cases, > 90% of the loaded bevacizumab was released and about 87% of the released antibody showed a binding comparable to fresh bevacizumab.

### Description of the Figures:

Fig. 1 shows the 4arm and 8arm poloxamines provided with furyl (Fig. 1A) and maleimide (Fig. 1B) groups as preferred types of polymer A and polymer B for use in the present invention.
Figure 2 shows the rheological behavior of 30 wt.% Poloxamine (Fig. 2A) and DA-Poloxamine (Fig. 2B) solutions in the course of a three-step temperature program. Absolute values of the complex shear modulus (|G*|), represented as white squares, and phase angles (δ), represented as black circles, were recorded as a function of temperature and time at a 0.5 Hz oscillatory frequency. The initial temperature was set to 20 °C. After 10 min, the temperature was increased to 37 °C (+ΔT) and kept constant for 180 min. In the third step, the temperature was again reduced to 20 °C (-ΔT).
Figure 3 shows the rheological behavior and gel formation of a 40% (w/V) 4armPoloxamine hydrogel at a constant temperature of 20 °C. Storage (G') and loss modulus (G") were recorded as a function of time. Both moduli increased as a result of covalent cross-linking via Diels-Alder reaction. After circa 257 minutes, the gel point was reached, i.e. the time-point after which the elastic properties of the gel were more dominant than the viscous properties (G' > G").
Figure 4 shows the initial thermal gelation and subsequent increase of stiffness for different 8armPoloxamine and 4armPoloxamine mixtures using an overall polymer concentration of 30 wt.%. Absolute values of the complex shear moduli (|G*|) were recorded over time at a 0.5 Hz oscillatory frequency. The initial temperature was set to 20 °C. After 10 min the temperature was increased to 37 °C (+ΔT) and then kept constant for 90 min.
Figure 5 shows the swelling and degradation of DA-Poloxamine hydrogels with different compositions in phosphate buffer, pH 7.4 at 37 °C (Fig. 5A). A higher number of functional end-groups correlated with an increase in stability and a lower degree of swelling. The onset of hydrogel erosion, i.e., beginning decrease of relative hydrogel mass, could be correlated with the concentration of 8armPoloxamine using a second-order polynomial (Fig. 5B).
Figure 6 shows the release of bevacizumab from 0%/30%, 5%/25%, and 10%/20% DA-Poloxamine hydrogels in phosphate buffer, pH 7.4 at 37 °C.

## Claims

1. A composition comprising a plurality of molecules of a polymer A and a plurality of molecules of a polymer B, wherein
polymer A carries two or more diene groups bound to the polymer structure as pendant reactive groups for a Diels-Alder reaction with polymer B;
polymer B carries two or more dienophile groups bound to the polymer structure as pendant reactive groups for a Diels-Alder reaction with polymer A;
and wherein at least one of polymer A and polymer B is a temperature-responsive polymer.

2. The composition according to claim 1, wherein at least one of polymer A and polymer B carries three or more of the reactive groups for a Diels-Alder reaction.

3. The composition according to claim 1 or 2, wherein the diene groups bound to polymer A are selected from a furane moiety, a cyclopentadiene moiety and a cyclohexadiene moiety, or from combinations thereof.

4. The composition according to any of claims 1 to 3, wherein the dienophile groups bound to polymer B are selected from a maleic anhydride moiety, a maleimide moiety, and a 1,4-quinone moiety, ore from combinations thereof.

5. The composition according to any of claims 1 to 4, wherein the temperature-responsive polymer has a gel formation temperature in water in the range of 25 to 41 °C, more preferably 30 to 41 °C, and even more preferably of 32 to 39 °C.

6. The composition according to any of claims 1 to 5, wherein polymer A and B are both a temperature responsive polymer.

7. The composition according to any of claims 1 to 6, wherein the temperature-responsive polymer has a block copolymer structure comprising one or more poly(ethylene glycol) blocks as blocks of a hydrophilic polymer, and one or more poly(propylene glycol) blocks as blocks of a hydrophilic polymer.

8. The composition according to claim 7, wherein each of polymer A and polymer B has a branched poly(ethylene glycol) - poly(propylene glycol) block copolymer structure wherein n block copolymer chains, each formed by a poly(propylene glycol) block and a poly(ethylene glycol) block, extend from a n-valent branching moiety, and wherein n is an integer of 3 to 8.

9. The composition according to any of claims 1 to 8, wherein polymer A and polymer B are dissolved or dispersed in a liquid carrier comprising water.

10. The composition according to any of claims 1 to 9 which is a pharmaceutical composition comprising one or more therapeutically active agents.

11. The composition according to claim 10, wherein the therapeutically active agent is a peptide or a protein.

12. A hydrogel which is obtainable via the gelation of a composition in accordance with any of claims 1 to 11, said gelation involving the thermal gelation of the composition comprising a plurality of molecules of a polymer A and a plurality of molecules of a polymer B, and the Diels-Alder reaction of the diene reactive groups of polymer A with the dienophile reactive groups of polymer B.

13. A kit of parts comprising
a plurality of molecules of a polymer A and a plurality of molecules of a polymer B, wherein
polymer A carries two or more diene groups bound to the polymer structure as pendant reactive groups for a Diels-Alder reaction with polymer B;
polymer B carries two or more dienophile groups bound to the polymer structure as pendant reactive groups for a Diels-Alder reaction with polymer A;
and wherein at least one of polymer A and polymer B is a temperature-responsive polymer.

14. The composition in accordance with any of claims 1 to 12 for use in therapy.

15. A method for the preparation of a composition in accordance with any of claims 9 to 11 wherein polymer A and polymer B are dissolved or dispersed in a liquid carrier comprising water, said method comprising the steps of:
obtaining the polymer A and the polymer B, and dissolving or dispersing the polymer A and the polymer B in a common liquid carrier comprising water to provide a solution or dispersion comprising a mixture of polymer A and polymer B; or
obtaining the polymer A and the polymer B, dissolving or dispersing the polymer A and the polymer B in separate liquid carriers comprising water to provide a solution or dispersion comprising polymer A and a solution or dispersion comprising polymer B and mixing these solutions or dispersions to provide a solution or dispersion comprising a mixture of polymer A and polymer B.
